(19) Europäisches Patentamt
European Patent Office
Office européen des brevets

(11) Veröffentlichungsnummer: **0 083 105**
**B1**

(12) # EUROPÄISCHE PATENTSCHRIFT

(45) Veröffentlichungstag der Patentschrift: **26.11.86**

(51) Int. Cl.⁴: **C 07 C 103/187,** C 07 C 102/00

(21) Anmeldenummer: **82112061.5**

(22) Anmeldetag: **28.12.82**

(54) **Verfahren zur kontinuierlichen Herstellung von 3-Aminocrotonsäureamid.**

(30) Priorität: **29.12.81 DE 3151709**

(43) Veröffentlichungstag der Anmeldung: **06.07.83 Patentblatt 83/27**

(45) Bekanntmachung des Hinweises auf die Patenterteilung: **26.11.86 Patentblatt 86/48**

(84) Benannte Vertragsstaaten: **CH DE GB LI**

(56) Entgegenhaltungen: **US-A-4 093 654**

(73) Patentinhaber: **WACKER- CHEMIE GMBH, Prinzregentenstrasse 22, D-8000 München 22 (DE)**

(72) Erfinder: **Künstle, Gerhard, Dr. Dipl.- Chem., Moosbrunnerstrasse 29, D-8263 Burghausen (DE)**
Erfinder: **Jung, Herbert, Bachstrasse 12, D-8263 Burghausen (DE)**

**EP 0 083 105 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

3-Aminocrotonsäureamid (ß-Aminocrotonamid, 3-Amino-buten(2)-säureamid) ist als wertvolles Zwischenprodukt für die Synthese von Pflanzenschutzmitteln, wie Phosphorsäureestern von substituierten Hydroxypyrimidinen, die beispielsweise unter der geschützten Bezeichnung "Diazinon" im Handel sind oder für die Herstellung von Stabilisatoren für Vinylverbindungen bekannt.

Die Herstellung von 3-Aminocrotonsäureamid durch Umsetzung von Diketen und Ammoniak in Gegenwart von Diethylether, die über die intermediäre Bildung von Acetoacetamid verläuft, gemäß der Gleichung:

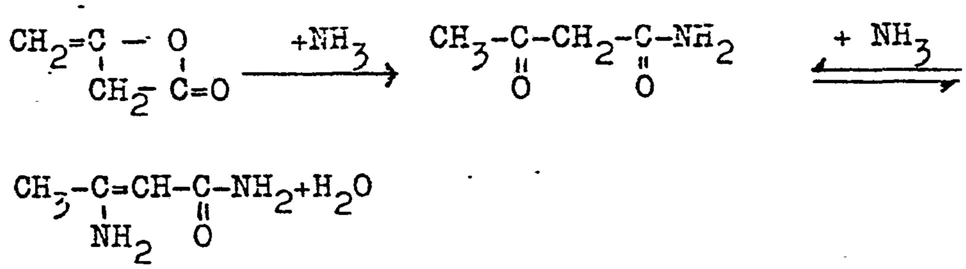

ist ebenfalls seit langem bekannt (vgl. J. Chem. Soc. 97 (1910), S. 1978 ff.).

Da aber offensichtlich die Isolierung des 3-Aminocroton-säureamids wegen seiner guten Löslichkeit und leichten Zersetzlichkeit in Wasser aus dem Wasser enthaltenden Reaktionsgemisch Schwierigkeiten bereitet, sind in der Zwischenzeit mehrere Verfahren bekannt geworden, die die Gewinnung des wasserfreien Endproduktes erleichtern sollten. So ist beispielsweise die Isolierung des 3-Aminocroton-säureamids aus dem Reaktionsgemisch durch Zugabe von Kaliumcarbonat für die Entfernung des Reaktionswassers und anschließender Extraktion mit Chloroform bekannt (vgl. JP-AS 68 02,686, referiert in C. A. 69, 105938q). Dieses Verfahren ist jedoch sehr umständlich, deshalb wurden anstelle von Diethylether andere Lösungsmittel verwendet, um die aufwendige Extraktionsbehandlung einzusparen. Beispiele für derartige Lösungsmittel sind m- oder p-Dioxan (vgl. JP-AS 71 08,686, referiert in C. A. 75, 35178q), aliphatische oder cycloaliphatische Kohlenwasserstoffe, aromatische Kohlenwasserstoffe, chlorierte Kohlenwasserstoffe und Dialkylether (vgl. JP-OS 72 18,826, referiert in C. A. 77, 151516p und DE-PS 20 37 388, die der US-PS 3 703 518 entspricht), acyclische Polyether (vgl. JP-OS 78 90,216, referiert in C. A. 90, 5936c) und Carbonsäureester (vgl. DE-0S 28 42 149). Die Umsetzung kann entweder in Gegenwart der Lösungsmittel durchgeführt werden oder diese werden nach beendeter Umsetzung zugefügt. Anschließend wird das Reaktionsprodukt aus der Lösung durch Abkühlen, gegebenenfalls unter Zusatz von Kristallisationskeimen ausgefällt und vom Lösungsmittel abgetrennt oder das Lösungsmittel wird als azeotropes Gemisch mit Wasser unter vermindertem Druck abdestilliert (vgl. JP-OS 78 9217, referiert in C. A. 90, 5935b).

Nach diesen bekannten Verfahren wird aber entweder kein reines 3-Aminocrotonsäureamid erhalten oder sie sind mit ungewöhnlich hohem Aufwand verbunden, da sie zusätzliche Regenerierungsmaßnahmen erfordern, die sowohl Ausbeuteverluste an dem gewünschten Endprodukt als auch Lösungsmittelverluste zur Folge haben.

Es stellt sich somit die Aufgabe, ein kontinuierliches Verfahren zur Herstellung von 3-Aminocrotonsäureamid durch Umsetzung von Diketen und Ammoniak in Abwesenheit von Lösungsmitteln zur Verfügung zu stellen, daß die Gewinnung der gewünschten wasserfreien Verbindung in hoher Ausbeute und Reinheit ermöglicht, ohne daß hierzu Lösungs- bzw. Verdünnungsmittel für die Ausfällung des Endproduktes oder Destillationshilfsmittel für die Entfernung des Reaktionswassers benötigt werden.

Diese Aufgabe wird erfindungsgemäß dadurch gelöst, daß die Umsetzung in flüssiger Phase bei Temperaturen von 40° bis 60° C in zwei Stufen durchgeführt wird, wobei in der ersten Stufe stöchiometrische Mengen von Diketen und Ammoniak gleichzeitig in das entstehende Reaktionsgemisch eingetragen, das Reaktionsgemisch nach einer mittleren Verweilzeit von 0,25 bis 1 Std. ausgetragen, dieses in der zweiten Stufe unter einem Ammoniak-Überdruck von 0,01 bis 0,2 bar 0,5 bis 5 Std. gehalten und anschließend das Reaktionsprodukt unter schonenden Bedingungen getrocknet wird.

Für die Durchführung des erfindungsgemäßen Verfahrens werden in der 1. Verfahrensstufe Diketen und Ammoniak definitionsgemäß im Molverhältnis 1:2 fortlaufend und gleichzeitig in das entstehende Reaktionsgemisch eingetragen, das neben 3-Aminocrotonsäureamid und Wasser geringe Mengen der Ausgangsstoffe und Acetoacet amid enthält. Entscheidend ist hierbei, daß die Umsetzung in flüssiger Phase erfolgt, d. h. bei Temperaturen von mindestens 40° C, da das entstehende Reaktionsgemisch bei Temperaturen < 40° C bereits zum Auskristallisieren neigt. Temperaturen von 60° C sollten jedoch nicht überschritten werden, da bei Temperaturen von > 60° C die Instabilität des 3-Aminocrotonsäureamids in dem Wasser enthaltenden Reaktionsgemisch zunimmt und die Löslichkeit von Ammoniak abnimmt.

Die Zusammensetzung des entstehenden Reaktionsgemisches ist dabei selbstverständlich nicht nur von der Temperatur, sondern auch vom Durchmischungsgrad und der Produktverweilzeit abhängig. Wenn für eine

möglichst rasche Durchmischung und damit gute Verteilung der Reaktionspartner gesorgt wird, sind mittlere Produktverweilzeiten von definitionsgemäß 0,25 bis 1 Std. für die Erzielung einer möglichst vollständigen Umsetzung des Diketens ausreichend. Da sich Reaktionstemperatur und Verweilzeit in Bezug auf die selektive Bildung von 3-Aminocrotonsäureamid umgekehrt proportional verhalten, sind Reaktionstemperaturen, die nur wenig, d. h. etwa 1° bis 10°C oberhalb des Schmelzbereiches der Reaktionsmischung liegen, bei einer mittleren Verweilzeit von 0,5 Std. besonders vorteilhaft.

In der 2. Verfahrensstufe, bei der das Reaktionsgemisch der unmittelbaren Einwirkung von Diketen entzogen ist wird dieses in Gegenwart von überschüssigem Ammoniak unter den gleichen Bedingungen in Bezug auf die Reaktionstemperatur, d. h. ebenfalls in flüssiger Phase definitionsgemäß 0,5 - 5 Std. gehalten, wobei sich eine mittlere Verweilzeit von 1 Stunde besonders bewährt hat, um sicherzustellen, daß das im Reaktionsgemisch noch vorhandene Acetoacetamid praktisch vollständig in 3-Aminocrotonsäure-amid übergeführt wird. Die Ammoniakatmosphäre wird dabei durch Einleiten von Ammoniak in das Reaktionsgemisch erzeugt, wobei gegebenenfalls durch mechanische Bewegung für eine gute Durchmischung gesorgt werden kann. Der Ammoniaküberdruck beträgt definitionsgemäß 0,01 - 0,2 bar, vorzugsweise 0,1 bar.

Unmittelbar anschließend an die 2. Verfahrensstufe wird das Reaktionsprodukt unter schonenden Bedingungen getrocknet, d. h. vom Reaktionswasser befreit. Die Trocknung kann in bekannter Weise bei Temperaturen von 30° - 100°C vorzugsweise von 40° - 60°C, vorgenommen werden, beispielsweise durch Vakuumtrocknung oder Sprühtrocknung unter Normaldruck (1,013 bar).

Die Durchführung des erfindungsgemäßen Verfahrens wird anhand einer bevorzugten Ausführungsform in Verbindung mit der Fig. wie folgt näher erläutert:

Die 1. Verfahrensstufe wird in einem Reaktionskreislauf (7) durchgeführt, der aus Umwälzpumpe (1), Wärmeaustauscher (2) und Abscheider (3) besteht und über Kühler (4) entlüftet ist. Über Leitung (5) werden Ammoniak in gasförmiger oder flüssiger Form und über Leitung (6) Diketen zugeführt. Gleichzeitig wird das bei Reaktionstemperatur im Kreislauf (7) befindliche Reaktionsgemisch über Leitung (8) im Maße seiner Bildung abgeführt und für die 2. Verfahrensstufe dem Nachverweilbehälter (9) zugeführt. Das Volumenverhältnis Betriebsinhalt (Nutzvolumen) zu Umlaufmenge im Kreislauf (7) kann dabei in weiten Grenzen variiert werden. So kann beispielsweise stündlich die 50 bis 500-fache Menge Reaktionsgemisch, bezogen auf den Betriebsinhalt im Kreislauf geführt werden. Die 2. Verfahrensstufe erfolgt im Nachverweilbehälter (9), dem über Leitung (10) Ammoniak zugeführt wird. Der Inhalt des Nachverweilbehälters (9) wird über Leitung (11) dem unter Normal. druck (1,013 bar) betriebenen Sprühtrockner (13) zugeführt und dort unter gleichzeitiger Zufuhr von Warmluft über Leitung (12), versprüht. Über Leitung (14) wird das Reaktionswasser zusammen mit der Warmluft ausgetragen und über Leitung (15) wird reines 3-Aminocrotonsäureamid ausgeschleust, das praktisch frei von Verunreinigungen ist und keiner weiteren Reinigung mehr bedarf.

Nach dem erfindungsgemäßen Verfahren kann somit 3-Aminocrotonsäureamid mit einer Reinheit von bis zu 99 % in hohen Ausbeuten, d. h. bis zu > 98 % der Theorie auf einfache und wirtschaftliche Weise in großtechnischem Maßstab hergestellt werden, und zwar ohne Mitverwendung von organischen Lösungsmitteln, die bisher zur Erzielung einer derartigen Reinheit unbedingt erforderlich waren.

Unter Berücksichtigung der großen Reaktionsfähigkeit der Ausgangsverbindungen, die unter den gegebenen Bedingungen zur Bildung zahlreicher Nebenprodukte fähig sind, der Instabilität des 3-Aminocrotonsäureamids in Gegenwart von Wasser, sowie der bekannten Schwierigkeiten bei der Wasserabetrennung für die Isolierung des wasserfreien Endproduktes, war es keineswegs vorhersehbar, daß durch Verzicht auf die Mitverwendung organischer Lösungsmittel bei dem erfindungsgemäßen Verfahren die Selektivität und Ausbeute gesteigert werden konnte.

In den folgenden Beispielen wird das erfindungsgemäße Verfahren näher erläutert:

**Beispiel 1**

(vgl, Figur)

Der verwendete Reaktionskreislauf (7) bestand aus einer Umwälzpumpe (1), einem mit Wasser gekühlten Spaltrohrwärmeaustauscher (2) und einem Abscheider (3), der über einen mit Wasser gekühlten Doppelrohrschlangenkühler (4) entlüftet war. Das Nutzvolumen (Betriebsinhalt) des Reaktionskreislaufs betrug (400 Vol. Tl. Der Nachverweilbehälter (9) bestand aus einem mit Warmwasser beheizten Rührwerkskessel, der über eine Tauchflasche (10) unter $NH_3$-Überdruck gehalten wurde.

Die Sprühtrocknung wurde in einem handelsüblichen Sprühtrockner (13) durchgeführt.

Dem Reaktionskreislauf (7) wurden über Leitung (6) stündlich 674 Gew. Tl.99,7 %iges, farbloses Diketen und über Leitung (5) 272 Gew. Tl. gasförmiges Ammoniak zugeführt. Während mit der Umwälzpumpe (1) stündlich 2 x $10^4$ Vol. Tl. Reaktionsgemisch, das neben molaren Mengen Wasser und 3-Aminocrotonsäureamid nur noch geringe Mengen Ausgangsstoffe und Acetoacetamid enthielt und dessen Schmelzpunkt 42°C betrug, bei einer Temperatur von 44°C im Kreislauf (7) geführt wurden, wurden gleichzeitig über den Siphonablauf (8) aus dem Abscheider (3) stündlich 946 Gew. Tl. Reaktionsgemisch ausgeschleust und adiabatisch dem Nachverweilbehälter (9) zugeführt. Das Nutzvolumen des Nachverweilbehälters (9), der unter einem $NH_3$-Überdruck von 0,1 bar bestand, betrug 2500 Vol. Tl. Von dort wurde das Reaktionsgemisch nach einer durchschnittlichen Verweilzeit von 1 Std. bei 44°C fortlaufend dem Sprühtrockner (13) zugeführt und unter

gleichzeitigem Einblasen von 3 x $10^4$ Vol. Tl. 44°C-warmer Luft je Vol. Tl. Reaktionsgemisch über Leitung (12) versprüht. Während über Leitung (14) das Reaktionswasser nahezu vollständig mit dem Luftstrom ausgetragen wurde, wurden über Leitung (15) stündlich 795 Gew. Tl. 99 %iges ($^1H$-NMR) 3-Aminocrotonsäureamid erhalten, das sind 98,3 % d. Th. Neben 0,3 % Wasser enthielt das erhaltene 3-Aminocrotonsäureamid noch 0,6 % Acetoacetamid.

Das gleiche Ergebnis wird erhalten, wenn Ammoniak in flüssiger Form eingesetzt wird.

Beispiele 2 mit 6:

Das Verfahren wurde unter Verwendung der gleichen Reaktionsapparatur, wie im Beispiel 1 beschrieben, wiederholt, jedoch unter Änderung von Temperatur und Verweilzeit. Die Reaktionsbedingungen, Ausbeuten und Analysenergebnisse sind in der folgenden Tabelle zusammengestellt:

| Beispiel Nr. | Reaktionsbedingungen: Reaktionskreislauf (7) Temperatur °C | Reaktionsbedingungen: Reaktionskreislauf (7) Verweilzeit h | Reaktionsbedingungen: Nachverweilbehälter (9) Verweilzeit h | Ausbeute in % d. Theorie, bezogen auf eingesetztes Diketen | Analysenergebnisse: 3-Aminocrotonsäureamid Gew. % lt. $^1H$-NMR-Analyse | Analysenergebnisse: Acetoacetamid Gew. % lt. $^1H$-NMR-Analyse | Analysenergebnisse: Wasser Gew. %, bestimmt nach der Methode von Karl Fischer |
|---|---|---|---|---|---|---|---|
| 2 | 42 | 0,25 | 2,5 | 98,0 | 98,6 | 0,7 | 0,4 |
| 3 | 50 | 0,5 | 2,5 | 97,7 | 98,3 | 1,0 | 0,2 |
| 4 | 60 | 0,25 | 2,5 | 97,5 | 98,1 | 1,0 | 0,3 |
| 5 | 60 | 1,0 | 1,0 | 96,6 | 96,4 | 2,5 | 0,3 |
| 6 | 42 | 0,5 | 0,5 | 97,3 | 97,6 | 1,8 | 0,2 |

## Patentansprüche

Verfahren zur kontinuierlichen Herstellung von 3-Amino-crotonsäureamid durch Umsetzung von Diketen und Ammoniak in Abwesenheit von Lösungsmitteln, dadurch gekennzeichnet, daß die Umsetzung in flüssiger Phase bei Temperaturen von 40° bis 60°C in zwei Stufen durchgeführt wird, wobei in der ersten Stufe stöchiometrische Mengen von Diketen und Ammoniak gleichzeitig in das entstehende Reaktionsgemisch eingetragen, das Reaktionsgemisch nach einer mittleren Verweilzeit von 0,25 bis 1 Std. ausgetragen, dieses in der zweiten Stufe in flüssiger Phase bei Temperaturen von 40° bis 60°C unter einem Ammoniak-Überdruck von 0,01 bis 0,2 bar 0,5 bis 5 Std. gehalten und anschließend das Reaktionsprodukt unter schonenden Bedingungen getrocknet wird.

## Claim

Process for the continuous manufacture of 3-aminocrotonic acid amide by reacting diketene and ammonia in the absence of solvents, characterised in that the reaction is carried out in the liquid phase at temperatures of from 40 to 60°C in two stages, wherein, in the first stage, stoichiometric quantities of diketene and ammonia are introduced simultaneously into the resulting reaction mixture, and the reaction mixture is discharged after an average dwell time of from 0.25 to 1 hour, and, in the second stage, the reaction mixture is maintained in a liquid phase at temperatures of from 40 to 60°C under an ammonia excess pressure of from 0.01 to 0.2 bar for from 0.5 to 5 hours and then the reaction product is dried under protecting conditions.

**Revendication**

Procédé pour préparer en continu l'amino-3 crotonamide par réaction du dicétène avec l'ammoniac en l'absence de solvants, procédé caractérisé en ce qu'on effectue la réaction en phase liquide, à des températures de 40 à 60° C, en deux étapes, au cours de la première de celles-ci on introduit simultanément des quantités stoechiométriques de dicétène et d'ammoniac dans le mélange réactionnel qui se forme, on fait sortir le mélange réactionnel après un temps de séjour moyen de 0,25 à 1 heure, on le maintient, au cours de la seconde étape, en phase liquide à des températures de 40 à 60° C sous une surpression d'ammoniac de 0,01 à 0,2 bar pendant 0,5 à 5 heures, et ensuite on sèche le produit réactionnel dans des conditions douces.

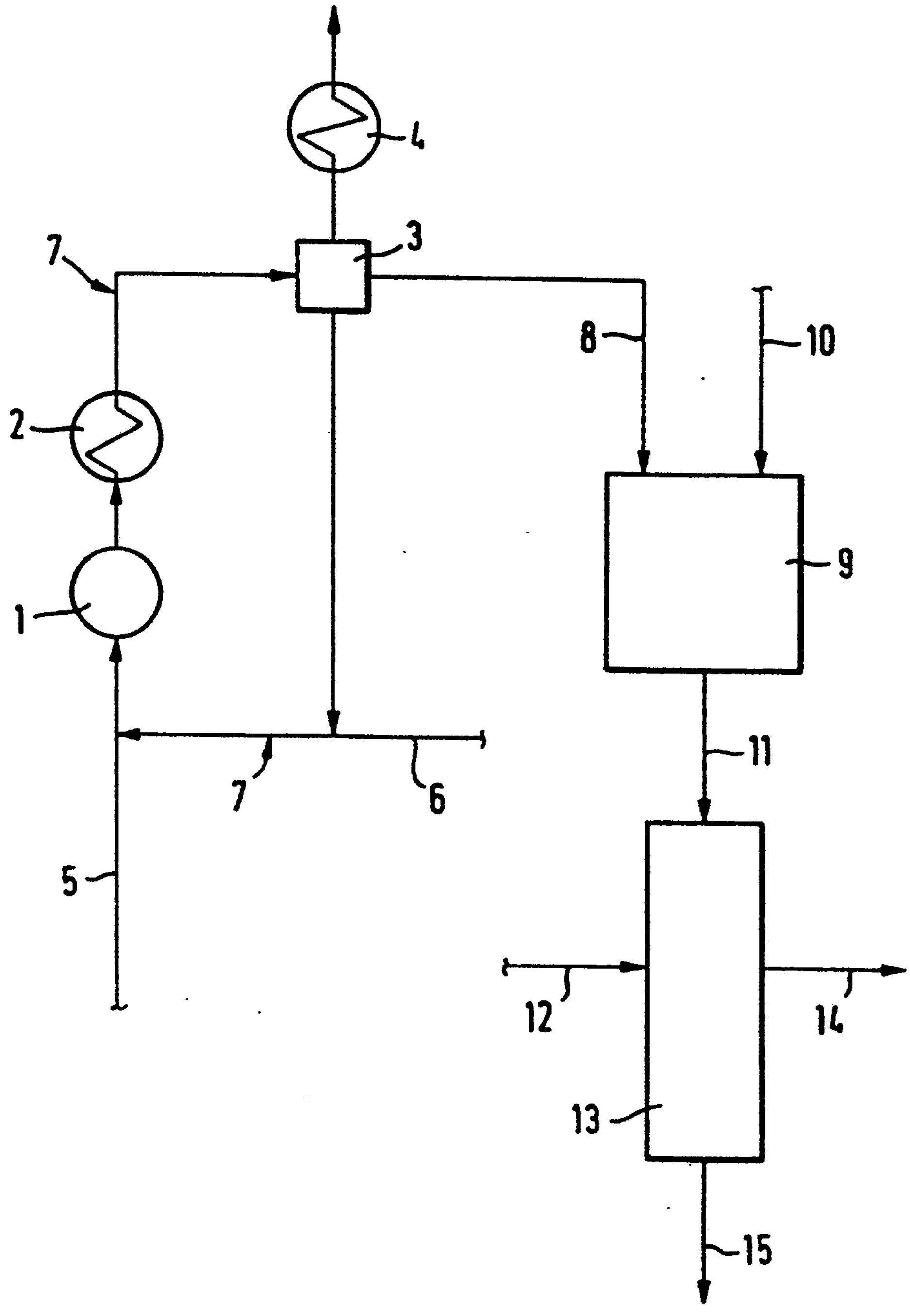

1
2
3
4
5
6
7
7
8
9
10
11
12
13
14
15